# EUROPEAN PATENT SPECIFICATION

(11) **EP 0 630 264 B1**
(45) Date of publication and mention of the grant of the patent: **06.11.2002**
(21) Application number: 93904905.2
(22) Date of filing: 05.02.1993
(51) Int. Cl.: A61K 49/00, A61K 45/00, C07F 13/00, C07K 5/08, C07K 5/02, C07D 213/64, C07D 405/12, C07C 323/60

(54) **LIGANDS FOR IMPROVING METAL CHELATE FORMATION KINETICS**
LIGANDEN ZUR VERBESSERUNG DER METALLCHELATBILDUNGSKINETIK
LIGANDS PERMETTANT D'AMELIORER LA CINETIQUE DE FORMATION D'AGENTS CHELATEURS

(30) Priority: 06.02.1992 US 832149; 25.02.1992 US 842017
(43) Date of publication of application: 28.12.1994
(73) Proprietor: BioSynthema Inc., St Charles, Missouri 63303 (US)
(72) Inventor: SRINIVASAN, Ananthachari, St. Charles, MO 63304 (US)
(74) Representative: Hooiveld, Arjen Jan Winfried
(86) International application number: US9301013
(87) International publication number: WO93015771

(56) References cited:
- WO-A-92/05154
- US-A- 4 638 051
- US-A- 4 963 688
- US-A- 5 071 636
- US-A- 5 075 099
- US-A- 5 095 111
- US-A- 5 202 451
- CHEMICAL ABSTRACTS, Volume 113, Number 12 (1990), Inorganic Chemistry, Vol. 29, No. 16, 8 August 1990, Easton, US, pages 2948-2951i, N. BRYSON et al., "Protecting Groups in the Preparation of Thiolate Complexes of Technetium".
- Bioconjugate Chemistry, Vol. 1, 1990, Easton, US, pages 132-137; K.E. BAIDOO et al., "Synthesis of a Diaminedithiol Bifunctional Chelating Agent for Incorporation of Technetium-99M into Biomolecules".
- Tetrahedron Letters, Vol. 32, No. 40, 1991, Great Britain, pages 5485-5488; L.M. GUSTAVSON et al., "Synthesis of a New Class of Tc Chelating Agents: N2S2 Monoaminemonoamide (MAMA) Ligands".

## Description

### FIELD OF THE INVENTION

This invention relates generally to novel amide-thiolate ligands having improved metal chelate formation kinetics. The amide-thiolate ligands of the present invention may be used for post formed labeling of biological substances useful in the fields of diagnosis and therapy.

### BACKGROUND OF THE INVENTION

Scintigraphic imaging and similar radiographic techniques for visualizing tissues in vivo are finding ever-increasing application in biological and medical research and in diagnostic and therapeutic procedures. Generally, scintigraphic procedures involve the preparation of radioactive agents which upon introduction to a biological subject, become localized in the specific organ, tissue or skeletal structure of choice. When so localized, traces, plots or scintiphotos depicting the in vivo distribution of radiographic material can be made by various radiation detectors, e.g., traversing scanners and scintillation cameras. The distribution and corresponding relative intensity of the detected radioactive material not only indicates the space occupied by the targeted tissue, but also indicates a presence of receptors, antigens, aberrations, pathological conditions, and the like.

In general, depending on the type of radionuclide and the target organ or tissue of interest, the compositions comprise a radionuclide, a carrier agent designed to target the specific organ or tissue site, various auxiliary agents which affix the radionuclide to the carrier, water or other delivery vehicles suitable for injection into, or aspiration by, the patient, such as physiological buffers, salts, and the like. The carrier agent attaches or complexes the radionuclide to the peptide carrier agent, which results in localizing the radionuclide being deposited in the location where the carrier agent concentrates in the biological subject.

Triamidethiolate and diamidedithiolate ligands have been used successfully for radiolabeling macromolecules. In general, amide-thiolate systems require harsh (75°C-100°C) radiolabeling conditions for preparing technetium and rhenium complexes. Under these conditions, the stability and biological properties of the small and medium bioactive peptides are often degraded.

In order to avoid harsh labeling conditions, pre-formed complexes have been coupled to the protein with some success. See Fritzberg et al., U.S. Patents Numbers 4,965,392 and 5,037,630 incorporated herein by reference. In the "pre-formed approach," the ligand is complexed with the radionuclide and then conjugated to the bioactive peptide. A major disadvantage of the pre-formed approach is that the end user must perform both the radiolabeling step and the coupling step (attaching the complex to the bioactive peptide). The final product must be purified prior to administration. In the case of small and medium sized peptides, the metal-complex may potentially react with "active sites" of the peptide. Thus, site specific attachment of a ligand to a bioactive molecule is only possible with post formed complexes.

In the conventional "post-formed approach," the ligand is first conjugated to the peptide and the resulting conjugate is labeled with the radioisotope under complex forming conditions. In the present invention, the post-formed approach has the additional advantage of allowing preparation of the conjugated bioactive peptide in kit form. The end users would perform only the radiolabeling step.

It has been found that the presence of free thiol (instead of protected thiol) and/or replacement of an amide with an amine causes labeling of N₂S₂ and N₃S ligands to proceed under milder conditions, but at the expense of some complex stability. See Rao et al., "Tc-Complexation of N₂S₂ Monoaminemonoamides," Int. J. Radial. Part B, (1991) (in press). In addition, Misra et al., "Synthesis of a Novel Diaminodithiol Ligand for Labeling Proteins and Small Molecules with Technetium-99m," Tetrahedron Letters, Vol. 30, No. 15, pp. 1885-88 (1989) and Baidoo et al., "Synthesis of a Diaminedithiol Bifunctional Chelating Agent for Incorporation of Technetium-99m into Biomolecules," Bioconjugate Chemistry, Vol. 1, pp. 132-37 (1990), report that diaminedithiol (DADT) ligands label with ^{99m}Tc at ambient temperatures.

Gustavson et al., "Synthesis of a New Class of Tc Chelating Agents: N₂S₂ Monoaminemonoamide (MAMA) Ligands," Tetrahedron Letters, Vol. 32, No. 40, pp. 5485-88 (1991), compares the radiolabeling efficiency of a N₂S₂-diamidedithiol (DADS) ligand with a N₂S₂-monoamine amide (MAMA) ligand. It was found that substitution of the amide nitrogen in the DADS ligand with an amine nitrogen in the MAMA ligand produced a threefold increase in radiochemical yield when labeling with ^{99m}Tc at 37°C for 30 minutes.

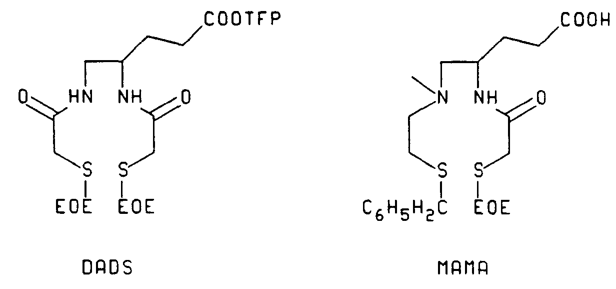

Notwithstanding the improved metal complex formation kinetics reported with amine-containing N₂S₂ and N₃S ligands, Tc and Re amide-thiolate complexes assure maximum in vivo stability and inhibit metal oxidation to the pertechnetate or perrhenate oxidation state.

It will be appreciated that it would be a significant advancement in the art to provide an amide-thiolate ligand with improved complex formation kinetics which can be labeled under mild conditions and which has excellent in vivo complex stability.

### SUMMARY OF THE INVENTION

The present invention discloses novel amide-thiolate ligands having improved complex formation kinetics. The present invention also includes radiolabeled peptide compounds utilizing the disclosed ligands, methods of preparing these compounds, pharmaceutical compositions comprising these compounds and the use of these compounds in kits for therapeutic and diagnostic applications.

Conventional amide-thiolate ligands, including N₃S and N₂S₂ ligands, are modified according to the present invention to include a center for formation of an amine-amide-thiolate kinetic intermediate complex, followed by transfer of the metal or conversion of the ligand into a thermodynamically stable amide-thiolate core.

In one aspect of the present invention, amide-thiolate ligands are modified by strategically locating an amine group to facilitate rapid formation of the amine-amide-thiolate intermediate complex. The "amine group" is preferably a tertiary amine, having an unshared pair of electrons capable of being donated to the metal electrophile during complex formation.

In order to form the amine-amide-thiolate intermediate complex, it is important that the amine be at a proper location and distance from the chelating core. For most applications, the amine group is separated from the carbon of the amino acid by two to five carbons, and preferably by three carbon atoms. The intermediate complex acts as a built-in metal transfer agent.

In another aspect of the present invention, the N₃S amide-thiolate ligands contain an amine within the N₃S core, to enhance initial complex formation kinetics, which converts to a thermodynamically stable amide during complex formation. The amine is preferably part of a pyridine ring containing a C_{1 to 4} alkoxyl substituent in the 2 or 4 position. O-dealkylation occurs upon complexation which causes the amine to become a vinylogous amide.

The amine-amide-thiolate complex formation and transfer of the metal to or ligand conversion to the amide-thiolate complex occurs under mild conditions. As used herein, the term "mild conditions" includes conditions of complex formation that do not adversely affect the targeting ability or biological activity of the carrier molecule. For most purposes, a complexing temperature in the range from about 25°C to about 50°C and a pH in the range from about 3-8 are sufficiently mild for small and medium peptides.

The amide-thiolate ligands within the scope of the present invention can be coupled as conjugates with biologically active molecules or biomolecules that are known to concentrate in the organ or tissues to be examined. These biomolecules include, for example, growth factors and synthetic analogs such as somatostatin, hormones such as insulin, prostaglandins, steroid hormones, amino sugars, peptides, proteins, lipids, etc. Conjugates with albumins, such as human serum albumin, antibodies, monoclonal antibodies specific to tumor associated antigens, or antimyosin, etc. The diagnostic media formed therefrom may be used in diagnostic and therapeutic applications.

In the present invention, the amide-thiolate ligand is coupled to the biomolecule according to standard procedures known in the art. In the case of small to medium peptides, the active sites of the biomolecules are protected so that the ligands are specifically attached to functional groups that are not involved in binding the biomolecules to the target receptor.

The ligands and biomolecule conjugates described above are useful in diagnostic and radiotherapy applications. The compounds of the present invention may be labeled with any suitable radionuclide favorable for these purposes. Such suitable radionuclides for radiotherapy include but are not limited to ¹⁸⁶Re, ¹⁸⁸Re, ⁶⁷Cu, ⁹⁰Y, and ⁶⁰Co. For diagnostic purposes the most suitable radionuclides include, but are not limited to, the transition metals as exemplified by ^{99m}To, ¹¹¹In, and ⁶²Cu.

It is therefore an object of the present invention to provide amide-thiolate ligands having improved complex formation kinetics which can be labeled under mild conditions and which have excellent complex stability.

### DETAILED DESCRIPTION OF THE INVENTION

The novel amide-thiolate ligands of the present invention are distinguished from conventional amide-thiolate ligands by having an amine group, or its equivalent, present for rapidly forming an amine-amide-thiolate intermediate complex. In one aspect of the present invention, the amine is present on a side chain which participates in the formation of an amine-amide-thiolate intermediate complex. The rapidly formed intermediate complex then transfers the metal to a thermddynamically stable amide-thiolate core. In another aspect of the present invention, the amine is present in the N₃S core and is rapidly converted to an amide upon complexation. The presence of the amine enhances the initial kinetics of chelate formation while the presence of the final amide provides a more thermodynamically stable amide-thiolate complex. Overall, the metal chelate formation kinetics are enhanced.

In one embodiment of the invention, the "amine group" is preferably a tertiary amine having an unshared pair of electrons capable of being donated to the metal electrophile during initial complex formation. Proper location and spacing of the amine group from the chelating core is necessary to form the desired amine-amide-thiolate intermediate complex. The amine group is preferably separated from the α carbon of the amino acid by two to five carbons, and most preferably by three carbon atoms.

Accordingly the invention provides a N₃S ligand having the general structure: 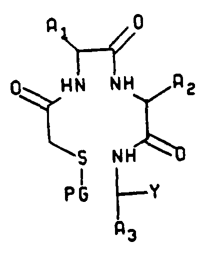 where at least A₁, A₂ or A₃ is -(CH₂)ₙNR₁R₂ and subject to the proviso that at least one of the remaining A₁, A₂ or A₃ is -(CH₂)_{n'}-X, and any remaining A₁, A₂, or A₃ is H or an alkyl or functionalized alkyl substituent of an α-amino acid; Y is selected from H, -(CH₂)_{n'}-X, and -(CH₂)_{n"}-COOH; X is a functional group for use in coupling the ligand to a bimolecule; R₁ and R₂ may be the same or different and are C₁₋₄ alkyl optionally containing a functional group X, with the proviso that if R₁ or R₂ contains X, or Y is -(CH₂)_{n'}-X, then the remaining A₁, A₂ or A₃ are H or an alkyl or functionalized alkyl substituent of an α-amino acid; n is from 2-5 with the proviso that when A₁ and A₂ are H, A₃ is -(CH₂)ₙNRCH₃, R is methyl or alkyl, and Y is -(CH₂)₃COOH, n may be 0 and when A₁ and A₂ are H, A₃ is -(CH₂)ₙNRCH₃, R is methyl or alkyl, and Y is -(CH₂)₂COOH, n may be 1; n' is from 1-10 and n" is from 0-4 with the proviso that when X is -COOH, n' is not 1-4; and PG is a sulfur protecting group.

The following generalized structures illustrate typical N₃S ligands containing amine groups coupled to side chains which can participate in the formation of intermediate complexes within the scope of the present invention. 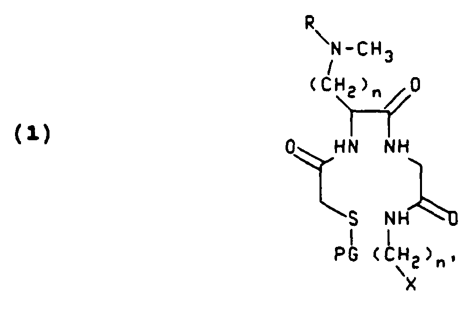 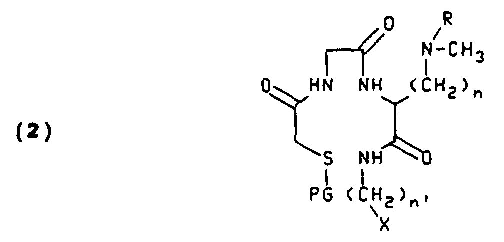 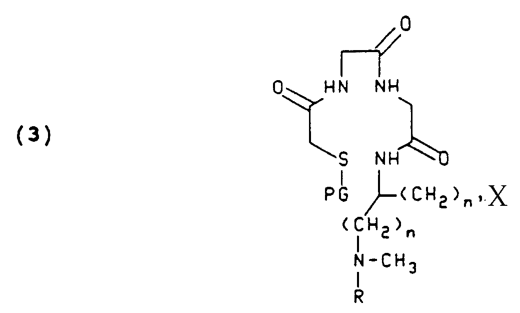 Where n is in the range from 2-5; n' is in the range from 1-10; X is a functional group capable of reacting with a biomolecule such as a carbonyl, active ester, isocyanate, isothiocyanate, imidate, maleimide or an activated electrophilic center such as C=C, halocarbonyl, halosulfonyl, and haloacetyl; R is methyl or alkyl groups optionally containing functional group X; and PG is a protecting group.

The protecting group prevents potential oxidation of the sulfur and prevents the sulfur from reacting with other reactive groups in the biologically active molecule during attachment of the ligand. The protecting group remains stable during kit formulation and stable until the metal (radioisotope) is added by the end user for conversion to the chelate. The protecting groups are removed concomitantly during complex formation, i.e., the protecting groups are removed only under labeling conditions and in the presence of the metal. Examples of typical protecting groups known in the art include hemithioacetal groups such as ethoxyethyl, methoxymethyl, substituted and unsubstituted tetrahydrofuranyl and tetrahydropyranyl, acetamidoalkyl such as actetamidomethyl, S-acyl such as S-alkanoyl, S-benzoyl, and S-substituted benzoyl groups.

The different side chain lengths can be prepared from modified dimethylamino amino acids; for n=2, from aspartic acid, for n=3, from glutamic acid, for n=3, from ornithine, and n=4, from lysine. The following reactions illustrate how the requisite dialkylamino compounds may be prepared: 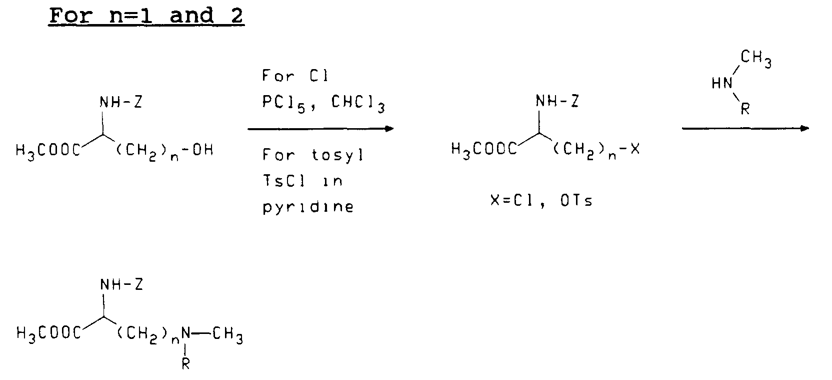 The requisite dialkylamino acid derivatives are prepared from commercially available Z-serine- and Z-threonine methyl ester (Z=benzyloxycarbonyl) derivatives. Dialkylamino groups are introduced by nucleophilic displacement of the Cl or the tosyl derivatives prepared according to the reaction shown below. (For conversion of serine and threonine derivatives to the chloro and tosyl derivatives, see A. Srinivasan, R. W. Stephenson and R. K. Olsen, J. Org. Chem., Vol. 42, p. 2256 (1977)). 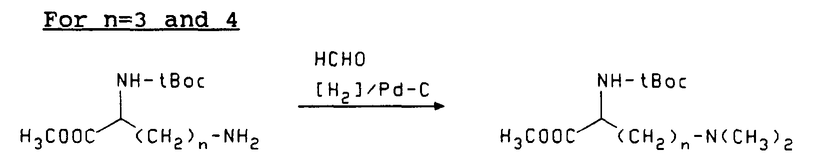 The starting materials are commercially available N-α-tBoc ornithine methyl ester, when n=3, and N-α-tBoc-lysine methyl ester, when n=4. The term "tBoc" is tertiary butoxy carbonyl. This methodology is well known in the art. 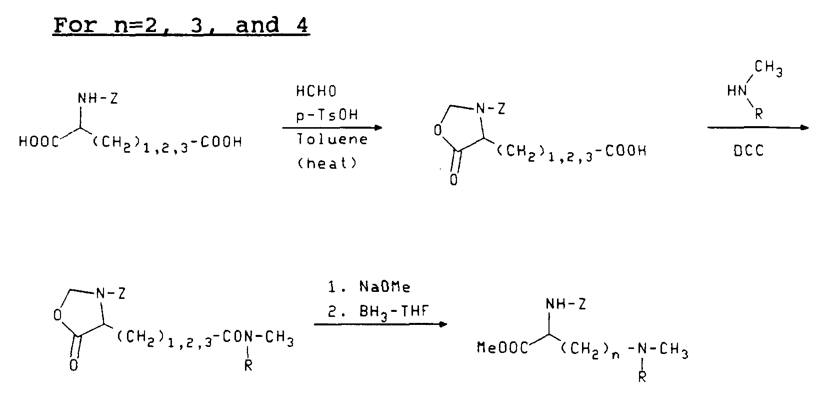 Commercially available N-Z-aspartic- (n=1), N-Z-glutamic-(n=2), and Z-aminoadipic (n=3) are converted to the oxazolidinones in the presence of p-formaldehyde and p-toluenesulfonic acid as the catalyst according to the procedure of R. Straka and M. Zaoral (Coll. of Czeck. Chem. Comm., Vol.42, p. 560 (1977)). The tertiary amide is prepared by DCC mediated condensation of the oxazolidinones (C. Itoh, Chem. Pharm. Bull., Vol. 17, p. 1679 (1969)). After the formation of methyl ester by sodium methoxide, the tertiary amide is reduced to the corresponding amine. (For borane reductions of tertiary amide to amine, see H. C. Brown and P. Heim, J. Org. Chem., Vol. 58, p. 912 (1978)). 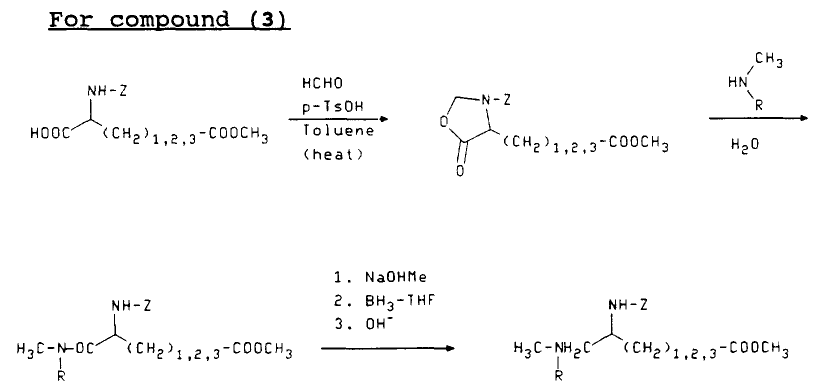 To prepare α-amides, the oxazolidinone distal methyl esters are reacted with requisite amine according to the procedure of K. Lee, et al., Synthesis, p. 931 (1991). Reduction of the α-amide to the amine is accomplished by borane reduction. (H. C. Brown and P. Heim, J. Org. Chem., Vol. 58, p. 912 (1978)).

The following generalized structure illustrates one possible N₂S₂ ligand containing an amine group coupled to a side chain which can participate in the formation of intermediate complexes within the scope of the present invention. 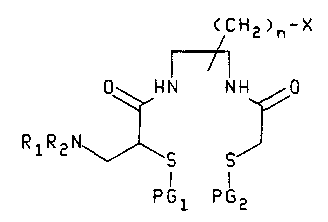 Where PG₁ and PG₂ may be the same or different sulfur protecting groups. At least one of PG₁ and PG₂ should be an acid labile protecting group selected from the group consisting of hemithioacetal groups such as ethoxyethyl and methoxymethyl, substituted and unsubstituted tetrahydrofuranyl and tetrahydropyranyl, acetamidoalkyl such as actetamidomethyl. X is a functional group capable of reacting with a biomolecule such as a carbonyl, active ester, isocyanate, isothiocyanate, imidate, maleimide or an activated electrophilic center such as C=C, halocarbonyl, halosulfonyl, and haloacetyl. R₁ and R₂ may be the same or different C₁₋₄ alkyl group.

In another embodiment within the scope of the present invention, the "amine group" is preferably part of a pyridine ring containing a C₁₋₄ alkoxyl substituent in the 2 or 4 position. O-dealkylation occurs upon complexation which causes the amine to become a vinylogous amide. Thus, the amine is a masked amide. In the claimed compounds, the amide necessary to form the chelate is masked as an amine by the presence of 2 or 4 alkoxyl substituent in the pyridine ring. Upon initial complex formation, O-dealkylation occurs to regenerate the amide.

The following generalized structure illustrates a typical N₃S ligands containing a masked amide group within the scope of the present invention. 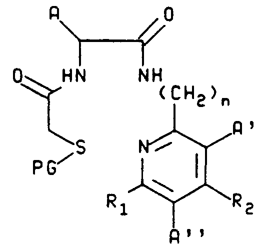 Where A is an H, alkyl, a functionalized substituent of an α-amino acid, or -(CH₂)_{n'}-X, where X is a functional group for coupling the ligand to a biomolecule, n' is from 1 to 10; R₁ or R₂ is a C₁₋₄ alkoxyl group, preferably methyoxyl, and the remaining R₁ or R₂ is H, alkyl, electron withdrawing group, or optionally -(CH₂)_{n'}-X if A is not -(CH₂)_{n'}-X; A' or A'' is H, alkyl, electron withdrawing group, or optionally -(CH₂)_{n'}-X if A, R₁, R₂ is not -(CH₂)_{n'}-X; n is 1 or 2; and PG is a protecting group, as described above. Examples of possible functional groups for coupling the ligand to a biomolecule include carbonyl, active ester, isocyanate, isothiocyanate, imidate, maleimide or an activated electrophilic center such as C=C, halocarbonyl, halosulfonyl, and haloacetyl. Electron withdrawing groups, such as carboxylic acid, are well known to those skilled in the art and include functional groups containing unsaturation or electronegative atoms, such as halogen.

The following examples are offered to further illustrate the formation of triamide-thiolate complexes via an amine-amide-thiolate kinetic intermediate complex within the scope of the present invention. These examples are intended to be purely exemplary and should not be viewed as a limitation on any claimed embodiment.

### Example 1

### Formation of Triamide-Thiolate complex for Compound (1)

The following diagram illustrates a mechanism for the formation of a triamide-thiolate complex for compound **(1)** identified above. In the diagram, 3N and S are planar, M=O forms a tetradentate complex, and n=3. The initial amine-amide-complex is a [5,7,5] system, which converts to a most preferred [5,5,5] triamide-thiolate system.

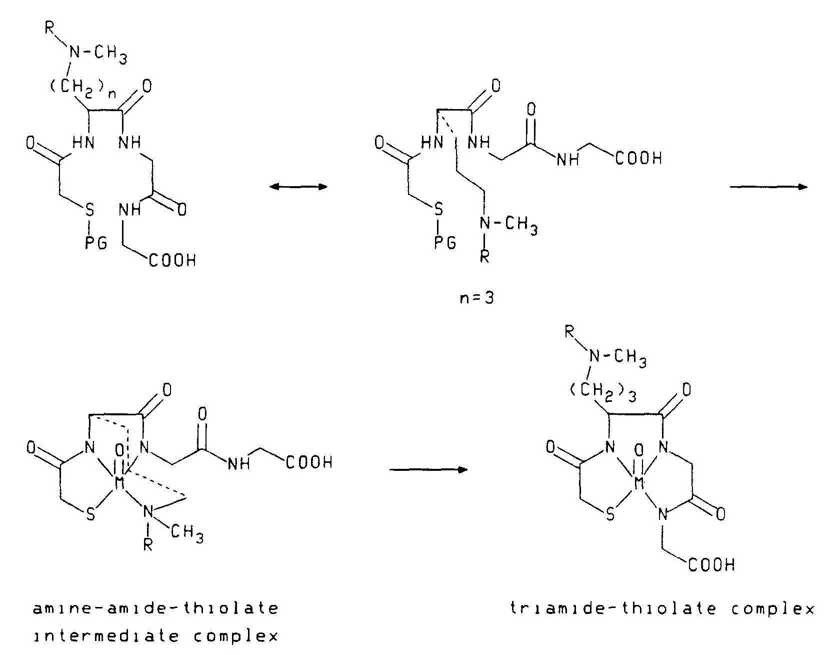

### Example 2

### Formation of Triamide-Thiolate complex for Compound (2)

The following diagram illustrates a mechanism for the formation of a triamide-thiolate complex for compound **(2)** identified above. In the diagram n=2. The initial amine-amide-complex is a [5,6,5] system, which converts to a most preferred [5,5,5] triamide-thiolate system. The M-N(R)-CH₃ is then displaced by the amide to form the triamide-thiolate complex. The more favorable ring size in the intermediate complex makes compound **(2)** preferable over compound **(1)**.

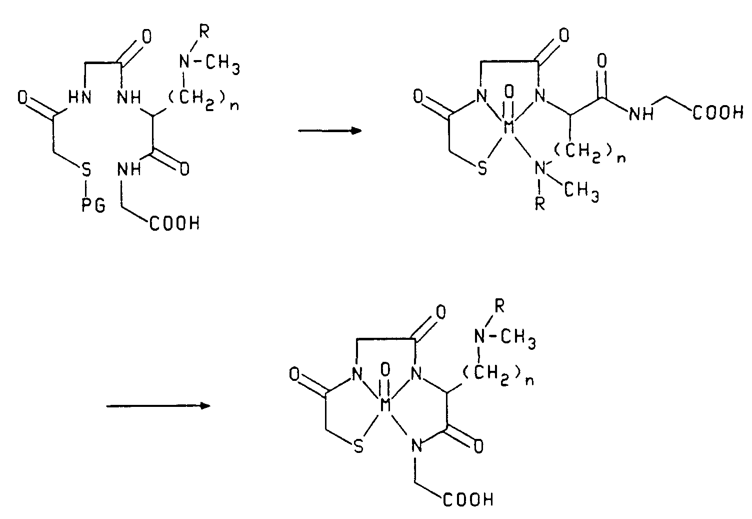

### Example 3

### Formation of Triamide-Thiolate Complex for Compound (3)

The following diagram illustrates a mechanism for the formation of a triamide-thiolate complex for compound **(3)** identified above having a terminal dialkylaminomethyl amino acid. Unlike compounds **(1)** and **(2)**, a transannular displacement mechanism in an eight membered intermediate complex leads to stale triamide-thiolate complex formation. Compound **(3)** is based on α-dimethylamino amino acids rather than tertiary amine containing α-amino acids. As described in Example 6, below, compound **(3)** may be prepared from commercially available aspartic and glutamic acid derivatives via oxazolidinones followed by reduction.

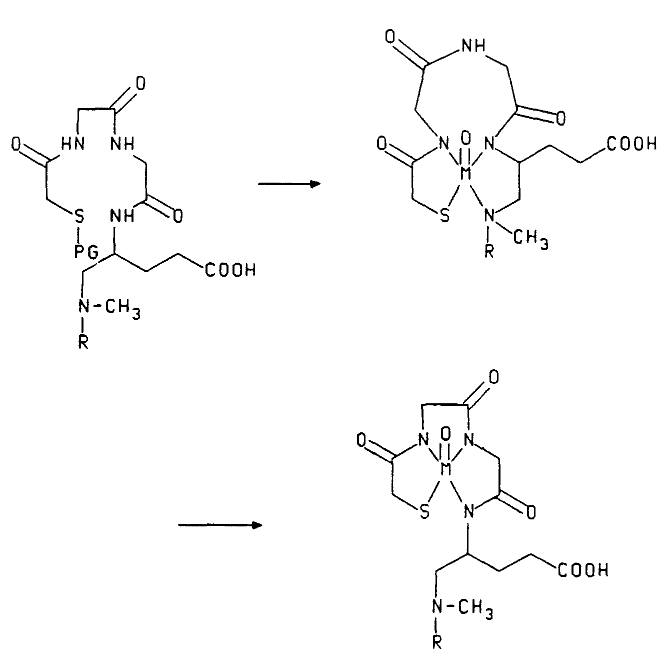

Since the amine-amide-thiolate intermediate complex acts as a built-in metal transfer agent, and since the intermediate complex forms under mild conditions, the overall formation kinetics of the amide-thiolate complex is improved.

The following examples are offered to further illustrate the synthesis of potential triamide-thiolate ligands within the scope of the present invention. These examples are intended to be purely exemplary and should not be viewed as a limitation on any claimed embodiment.

### Example 4

### Synthesis of Triamide-Thiolate Ligand, Compound (1)

A modified amino acid, described above, is reacted according to chemical reactions well known to those skilled in the art to yield compound (1). 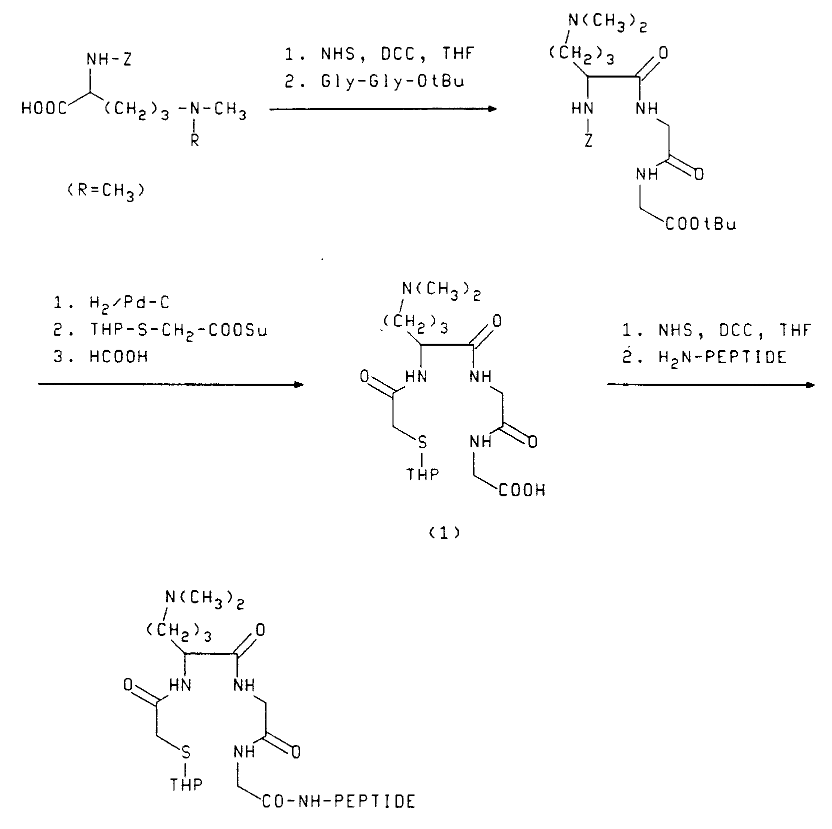 Where NHS is N-hydroxysuccinimide, Su is succinimide, and THP is 2-tetrahydropyranyl. As illustrated, the ligand may be coupled to a peptide according to techniques known in the art.

### Example 5

### Synthesis of Triamide-Thiolate Ligand, Compound (2)

A modified amino acid, described above, is reacted according to chemical reactions well known to those skilled in the art to yield compound (2). 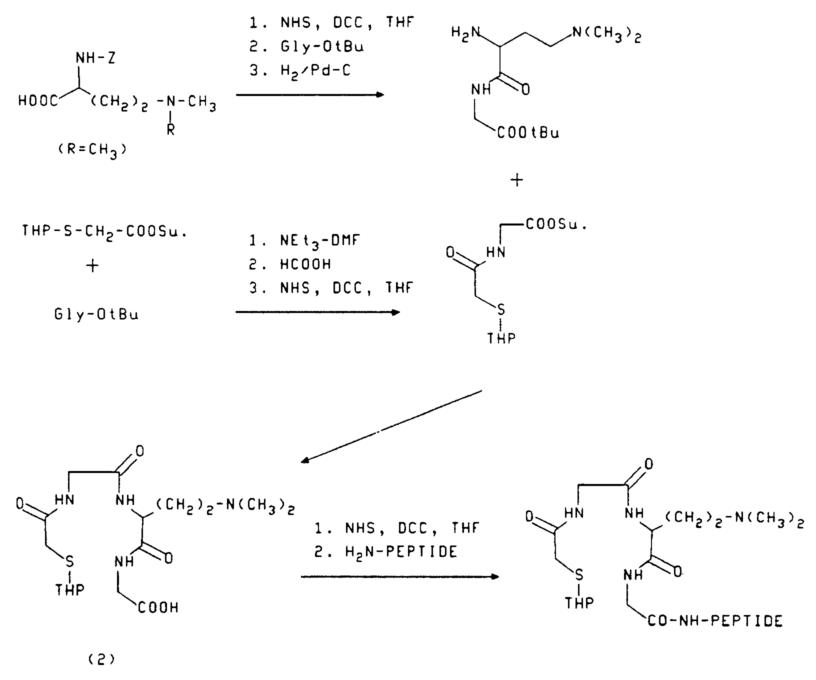 As illustrated, the ligand may be coupled to a peptide according to techniques known in the art.

### Example 6

### Synthesis of Triamide-Thiolate Ligand, Compound (3)

A modified amino acid, described above, is reacted according to chemical reactions well known to those skilled in the art to yield compound (3).

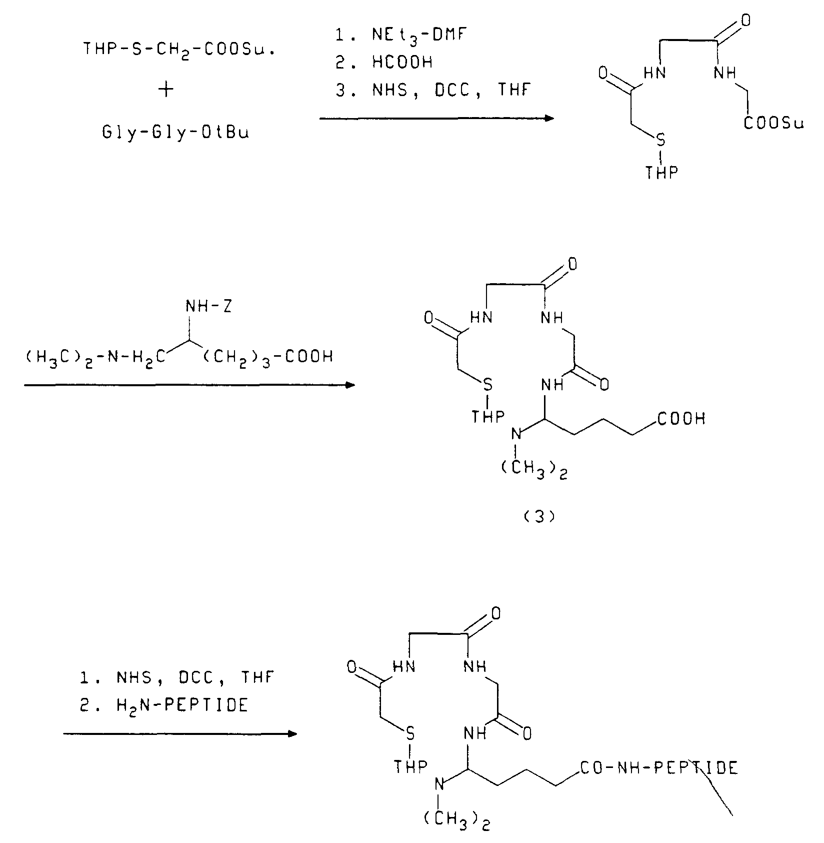

The following examples are offered to further illustrate methods of preparing various pyridine derivatives which may be used to prepare the ligands within the scope of the present invention. These examples are intended to be purely exemplary and should not be viewed as a limitation on any claimed embodiment.

### Example 7

The following diagram illustrates the synthesis of a pyridine derivative having a methoxyl substituent in the 2 position. Commercially available 2,6-dichloropyridine is converted to 2-cyano-6-methoxypyridine by successive nucleophilic substitution with cyanide and methoxide followed by catalytic reduction to give compound (A). 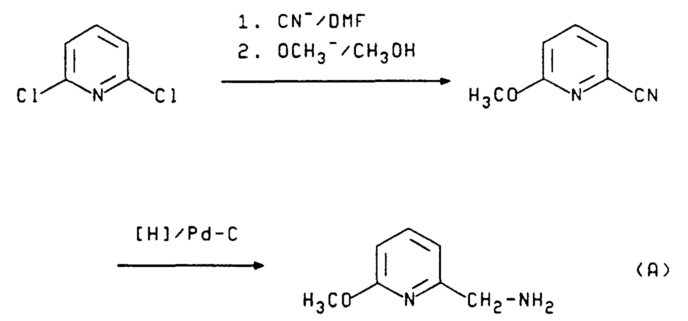 The final compound (A) may be used to prepare a ligand within the scope of the present invention in which n=1.

### Example 8

The following diagram illustrates the synthesis of a pyridine derivative having a methoxyl substituent in the 4 position. The initial 4-chloropyridine starting material is commercially available. The individual reactions are known to those skilled in the art. 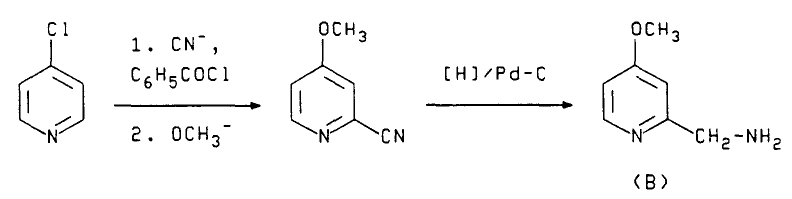 Support for the first step conversion of the chloro pyridine to the 2-cyano derivative is found in Yakugaku Zasshi, Vol. 65B, p. 582 (1945). The final compound (B) may be used to prepare a ligand within the scope of the present invention in which n=1.

### Example 9

The following diagram illustrates the synthesis of a pyridine derivative having a methoxyl substituent in the 2 position. Commercially available 2,6-dichloropyridine is converted to α-cyano-α-methylthio-6-methoxypyridine by successive nucleophilic substitution with the anion of methylthioacetonitrile and methoxide. Dethiation and reduction of the nitrile is accomplished with Ra-Ni in a single step to give compound (C). 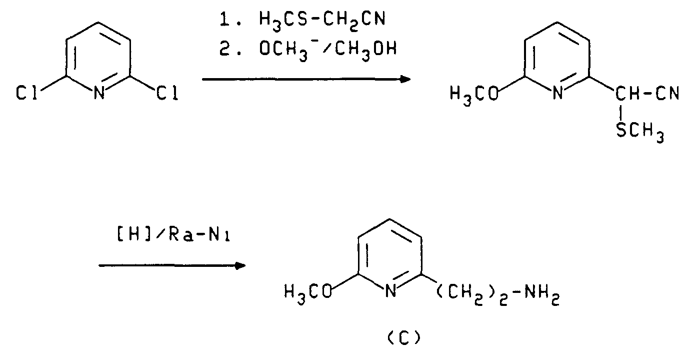 The final compound (C) may be used to prepare a ligand within the scope of the present invention in which n=2.

The following examples are offered to further illustrate the synthesis of potential triamide-thiolate ligands within the scope of the present invention. These examples are intended to be purely exemplary and should not be viewed as a limitation on any claimed embodiment.

### Example 10

In this example, compounds A (for n=1) or B (for n=2) are used as starting materials for the synthesis of ligands containing masked amides within the scope of the present invention. The other starting material, Z-glutamic acid γ-benzyl ester, is commercially available. The individual reactions are known to those skilled in the art. 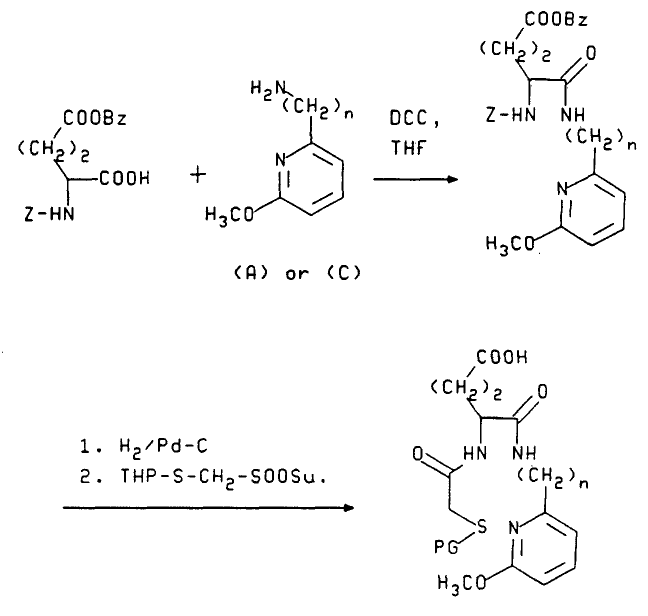 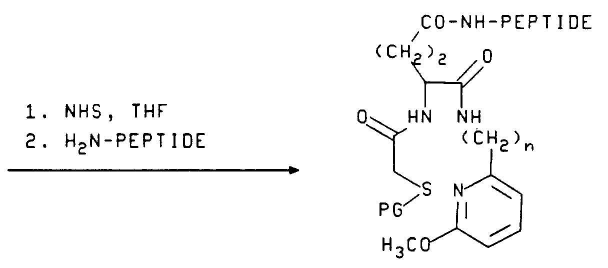 The same reaction conditions described above may be used when the alkoxyl group is in the 4 position (for compound B).

The quartenization of nitrogen heterocycles followed by O-dealkylation according to the Hilbert and Johnson reaction (alkoxyl group in the 2 position) reported in J. Amer. Chem. Soc., Vol. 52, p. 2001 (1930) as shown below: 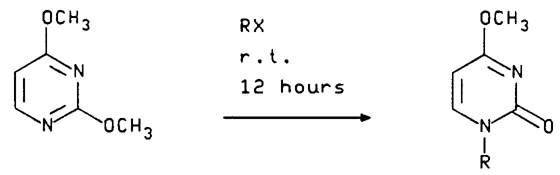 Where R is alkyl or acetal and X is a halide or anionic counter ion. Dealkylation of the alkoxyl group in the 4 position is reported by Fry et al., J. Chem. Soc., p. 5062 (1960) as shown below: 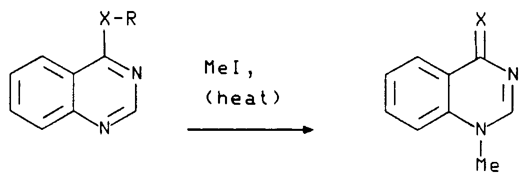 Where X is O or S, and when X is O, then R is phenyl and when X is S, then R is CH₃.

The ligands within the scope of the present invention are labeled according to standard labeling techniques. The following diagram illustrates the O-dealkylation and formation of a vinylogous amide for ligands in which the alkoxyl substituent is in the 2 position. 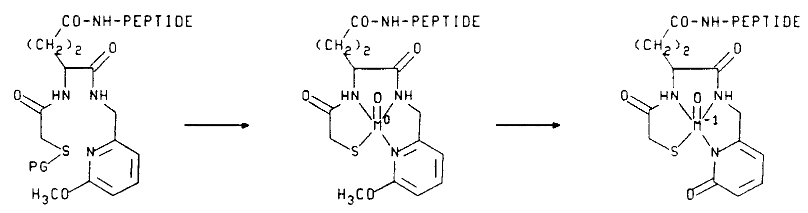 The following diagram illustrates the O-dealkylation and formation of a vinylogous amide for ligands in which the alkoxyl substituent is in the 4 position.

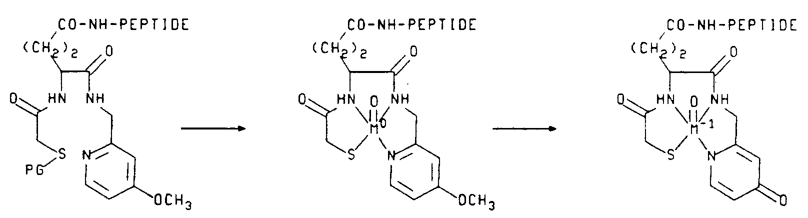

As described above, the ligands within the scope of the present invention may be coupled to biomolecules according to standard procedures known in the art. The conjugated biomolecules are then labeled with suitable radionuclides and administered to a patient for diagnostic imaging or therapeutic use.

After the amide-thiolate ligands of the present invention are prepared and labeled according to the procedure described above, the compounds may be used with a pharmaceutically acceptable carrier in conventional diagnostic imaging procedures. In this procedure, a diagnostically effective quantity of the compound, for example in the form of an injectable liquid, is administered to a warm-blooded animal and then imaged using a suitable detector, e.g. a gamma camera. Images are obtained by recording emitted radiation of tissue or the pathological process in which the radioactive peptide has been incorporated, which in the present care are tumors, thereby imaging at least a portion of the body of the warm-blooded animal.

Pharmaceutically acceptable carriers for either diagnostic or therapeutic use include those that are suitable for injection or administration such as aqueous buffer solutions, e.g. tris (hydroxymethyl)aminomethane (and its salts), phosphate, citrate, bicarbonate, etc., sterile water for injection, physiological saline, and balanced ionic solutions containing chloride and/or bicarbonate salts of normal blood plasma cations such as Ca²⁺, Na⁺, K⁺ and Mg²⁺. Other buffer solutions are described in Remington's Practice of Pharmacy, 11th edition, for example on page 170. The carriers may contain a chelating agent, e.g. a small amount of ethylenediaminetetraacetic acid, calcium disodium salt, or other pharmaceutically acceptable chelating agents.

The concentration of labeled biomolecule and the pharmaceutically acceptable carrier, for example in an aqueous medium, varies with the particular field of use. A sufficient amount is present in the pharmaceutically acceptable carrier in the present invention when satisfactory visualization of the tumor is achievable or therapeutic results are achievable.

From the foregoing, it will be appreciated that the present invention provides amide-thiolate ligands having improved complex formation kinetics which can be labeled under mild conditions and which have excellent complex stability.

The invention may be embodied in other specific forms without departing from its spirit or essential characteristics. The described embodiments are to be considered in all respects only as illustrative and not restrictive. The scope of the invention is, therefore, indicated by the appended claims rather than by the foregoing description. All changes which come within the meaning and range of equivalency of the claims are to be embraced within their scope.

## Claims

1. A N₃S ligand having the general structure: 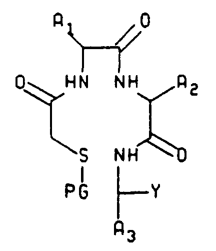 where at least A₁, A₂ or A₃ is -(CH₂)ₙNR₁R₂ and subject to the proviso that at least one of the remaining A₁, A₂ or A₃ is -(CH₂)_{n'}-X, and any remaining A₁, A₂, or A₃ is H or an alkyl or functionalized alkyl substituent of an α-amino acid; Y is selected from H, -(CH₂)_{n'}-X, and -(CH₂)_{n"}-COOH; X is a functional group for use in coupling the ligand to a bimolecule; R₁ and R₂ may be the same or different and are C₁₋₄ alkyl optionally containing a functional group X, with the proviso that if R₁ or R₂ contains X, or Y is -(CH₂)_{n'}-X, then the remaining A₁, A₂ or A₃ are H or an alkyl or functionalized alkyl substituent of an α-amino acid; n is from 2-5 with the proviso that when A₁ and A₂ are H, A₃ is -(CH₂)ₙNRCH₃, R is methyl or alkyl, and Y is -(CH₂)₃COOH, n may be 0 and when A₁ and A₂ are H, A₃ is -(CH₂)ₙNRCH₃, R is methyl or alkyl, and Y is -(CH₂)₂COOH, n may be 1; n' is from 1-10 and n" is from 0-4 with the proviso that when X is -COOH, n' is not 1-4; and PG is a sulfur protecting group.

2. A N₂S₂ ligand having the general structure: 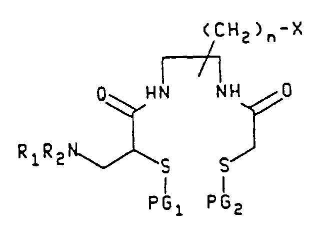 where X is a functional group for use in coupling the ligand to a biomolecule; n is from 2-5; R₁ and R₂ may be the same or different C₁₋₄ alkyl group; and PG₁ and PG₂ may be the same or different and are sulfur protecting groups, at least PG₁ or PG₂ being acid labile.

3. A N₃S ligand having the general structure: 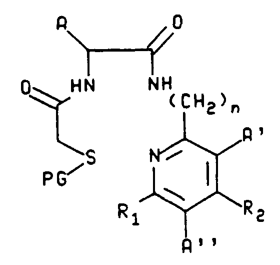 where A is H, an alkyl or functionalized alkyl substituent of an α-amino acid, or -(CH₂)_{n'}-X, where X is a functional group for use in coupling the ligand to a biomolecule and n' is from 1 to 10; R₁ or R₂ is a C₁₋₄ alkoxyl group, and the remaining R₁ or R₂ is H, alkyl, electron withdrawing group, or optionally -(CH₂)_{n'}-X if A is not -(CH₂)_{n'}-X; A' or A" is H, alkyl, electron withdrawing group, or optionally -(CH₂)_{n'}-X if A, R₁, or R₂ is not -(CH₂)_{n'}-X; n is 1 or 2; and PG is a sulfur protecting group.

4. A ligand as defined in any one of claims 1 to 3, wherein X is selected from the group consisting of a carbonyl, active ester, isocyanate, isothiocyanate, imidate, maleimide or an activated electrophilic center such as C=C, halocarbonyl, halosulfonyl, and haloacetyl.

5. A ligand as defined in any one of claims 1 to 4, wherein PG or PG₁ and/or PG₂ is/are selected from the group consisting of ethoxyethyl, methoxymethyl, substituted and unsubstituted tetrahydrofuranyl and tetrahydropyranyl, acetamidoalkyl, and S-acyl such as S-alkanoyl, S-benzoyl, and S-substituted benzoyl groups.

6. A ligand according to any one of claims 1 to 5 coupled to a biomolecule.

7. A diagnostic composition suitable for administration to a warm-blooded animal comprising:
a diagnostically effective quantity of a ligand according to any one of claims 1 to 5, wherein the ligand is coupled to a biomolecule and wherein the ligand is complexed with a radionuclide selected from ^{99m}Tc, ¹¹¹In, and ⁶²Cu; and
a pharmaceutically acceptable carrier.

8. A therapeutic composition suitable for administration to a warm-blooded animal comprising:
a therapeutically effective quantity of a ligand according to any one of claims 1 to 5, wherein the ligand is coupled to a biomolecule and wherein the ligand is complexed with a radionuclide selected from ¹⁸⁶Re, ¹⁸⁸Re, ⁶⁷Cu, ⁹⁰Y, and ⁶⁰Co; and
a pharmaceutically acceptable carrier.

9. A diagnostic or therapeutic kit comprising a coupled ligand according to claim 6; and a radionuclide.

## Patentansprüche

1. N₃S-Ligand mit der allgemeinen Struktur: 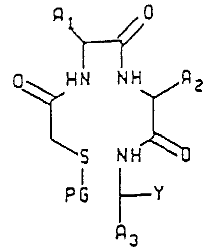 wobei wenigstens A₁, A₂ oder A₃ -(CH₂)ₙNR₁R₂ ist und unter der Bedingung, daß wenigstens eines der verbleibenden A₂, A₂ oder A₃ -(CH₂)_{n'}-X ist, und jedes verbleibende A₁, A₂ oder A₃ H oder ein Alkyl- oder funktionalisierter Alkylsubstituent einer α-Aminosäure ist; Y ausgewählt ist aus H, -(CH₂)_{n'}-X, und -(CH₂)_{n"}-COOH; X eine funktionelle Gruppe zur Verwendung beim Koppeln des Liganden an ein Bimolekül ist; R₁ und R₂ dieselben oder unterschiedlich sein können und C₁₋₄-Alkyl sind, das fakultativ eine funktionelle Gruppe X enthält, unter der Bedingung, daß, wenn R₁ oder R₂ X enthält oder Y -(CH₂)_{n'}-X ist, dann sind die verbleibenden A₁, A₂ oder A₃ H oder ein Alkyl- oder funktionalisierter Alkylsubstituent einer α-Aminosäure; n von 2-5 ist, unter der Bedingung, daß, wenn A₁ und A₂ H sind, A₃ -(CH₂)ₙNRCH₃ ist, R Methyl oder Alkyl ist, und Y -(CH₂)₃COOH ist, n 0 sein kann, und wenn A₁ und A₂ H sind, A₃ -(CH₂)ₙNRCH₃ ist, R Methyl oder Alkyl ist, und Y -(CH₂)₂COOH ist, n 1 sein kann; n' von 1-10 ist und n" von 0-4 ist, unter der Bedingung, daß, wenn X -COOH ist, n' nicht 1-4 ist; und PG eine Schwefelschutzgruppe ist.

2. N₂S₂-Ligand mit der allgemeinen Struktur: 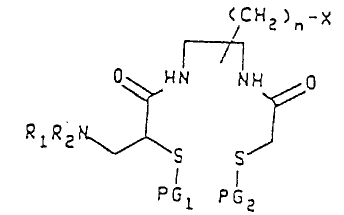 wobei X eine funktionelle Gruppe zur Verwendung beim Koppeln des Liganden an ein Biomolekül ist; n von 2-5 ist; R₁ und R₂ dieselbe oder eine unterschiedliche C₁₋₄-Alkylgruppe sein können; und PG₁ und PG₂ dieselben oder unterschiedlich sein können und Schwefelschutzgruppen sind, wobei wenigstens PG₁ oder PG₂ säurelabil ist.

3. N₃S-Ligand mit der allgemeinen Struktur: 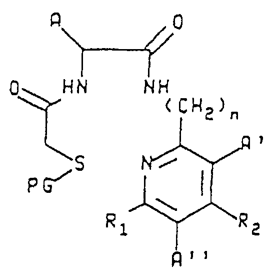 wobei A H, ein Alkyl- oder funktionalisierter Alkylsubstituent einer α-Aminosäure oder -(CH₂)_{n'}-X ist, wobei X eine funktionelle Gruppe zur Verwendung beim Koppeln des Liganden an ein Biomolekül ist, und n' von 1 bis 10 ist; R₁ oder R₂ eine C₁₋₄-Alkoxylgruppe ist, und der verbleibende R₁ oder R₂ H, Alkyl, eine elektronenziehende Gruppe oder fakultativ -(CH₂)_{n'}-X ist, wenn A nicht -(CH₂)_{n'}-X ist; A' oder A" H, Alkyl, eine elektronenziehende Gruppe oder fakultativ -(CH₂)_{n'}-X ist, wenn A, R₁, R₂ nicht -(CH₂)_{n'}-X ist; n 1 oder 2 ist; und PG eine Schwefelschutzgruppe ist.

4. Ligand, definiert nach einem der Ansprüche 1-3, wobei X ausgewählt ist aus der Gruppe, bestehend aus einem Carbonyl-, aktiven Ester-, Isocyanat-, Isothiocyanat-, Imidat-, Maleimid- oder ein aktiviertes elektrophiles Zentrum ist, wie C=C, Halogencarbonyl, Halogensulfonyl und Halogenacetyl.

5. Ligand, definiert nach einem der Ansprüche 1-4, wobei PG oder PG₁ und/oder PG₂ ausgewählt ist/sind aus der Gruppe, bestehend aus Ethoxyethyl, Methoxymethyl, substituiertem und nicht-substituiertem Tetrahydrofuranyl und Tetrahydropyranyl, Acetamidoalkyl und S-Acyl, wie S-Alkanoyl, S-Benzoyl und S-substituierte Benzoylgruppen.

6. Ligand nach einem der Ansprüche 1-5, gekoppelt an ein Biomolekül.

7. Diagnostische Zusammensetzung, geeignet zur Verabreichung an ein warmblütiges Tier, umfassend:
eine diagnostisch wirksame Menge eines Liganden nach einem der Ansprüche 1-5, wobei der Ligand an ein Biomolekül gekoppelt ist, und wobei der Ligand mit einem Radionuklid komplexiert ist, ausgewählt aus ^{99m}Tc, ¹¹¹In und ⁶²Cu; und
einen pharmazeutisch akzeptablen Träger.

8. Therapeutische Zusammensetzung, geeignet zur Verabreichung an ein warmblütiges Tier, umfassend:
eine therapeutisch wirksame Menge eines Liganden nach einem der Ansprüche 1-5, wobei der Ligand an ein Biomolekül gekoppelt ist, und wobei der Ligand mit einem Radionuklid komplexiert ist, ausgewählt aus ¹⁸⁶Re, ¹⁸⁸Re, ⁶⁷Cu, ⁹⁰Y und ⁶⁰Co; und
einen pharmazeutisch akzeptablen Träger.

9. Diagnostischer oder therapeutischer Kit, umfassend einen gekoppelten Liganden nach Anspruch 6; und ein Radionuklid.

## Revendications

1. Un ligand N₃S ayant la structure générale : 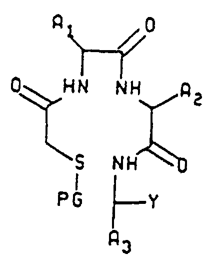 où au moins A₁, A₂ ou A₃ est -(CH₂)ₙNR₁R₂ et sous la condition que au moins l'un des A₁, A₂ ou A₃ restants est -(CH₂)_{n'}-X, et tout A₁, A₂ ou A₃ restant est H ou un alkyle ou un substituant alkyle fonctionnalisé d'un acide α-amino ; Y est choisi parmi H, -(CH₂)_{n'}-X, et -(CH₂)_{n"}-COOH ; X est un groupe fonctionnel destiné au couplage du ligand à une biomolécule ; R₁ et R₂ peuvent être les mêmes ou différents et sont des alkyles C₁₋₄ contenant éventuellement un groupe fonctionnel X, sous la condition que si R₁ et R₂ contient X, ou Y est -(CH₂)_{n'}-X, alors les A₁, A₂ ou A₃ restants sont H ou un alkyle ou un substituant alkyle fonctionnalisé d'un acide α-amino ; n est compris entre 2 et 5 sous la condition que lorsque A₁ et A₂ sont H, A₃ est -(CH₂)ₙNRCH₃, R est un méthyle ou un alkyle, et Y est -(CH₂)ₙCOOH, n peut être 0 et lorsque A₁ et A₂ sont H, A₃ est -(CH₂)ₙNRCH₃, R est un méthyle ou un alkyle, et Y est -(CH₂)₂COOH, n peut être 1 ; n' est compris entre 1 et 10 et n" est compris entre 0 et 4 sous la condition que lorsque X est -COOH, n' n'est pas compris entre 1 et 4 ; et PG est un groupe soufre protecteur.

2. Un ligand N₂S₂ ayant la structure générale 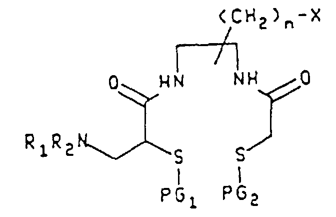 où X est un groupe fonctionnel destiné au couplage du ligand à une biomolécule ; n est compris entre 2 et 5 ; R₁ et R₂ peuvent être des groupes alkyles C₁₋₄ les mêmes ou différents ; et PG₁ et PG₂ peuvent être les mêmes ou différents et sont des groupes soufre protecteurs, au moins PG₁ ou PG₂ étant un acide labile.

3. Un ligand N₃S ayant la structure générale 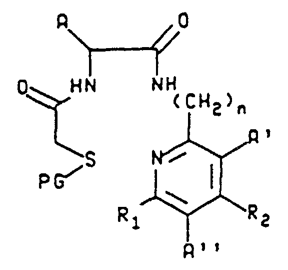 où A est H, un alkyle ou un substituant alkyle fonctionnalisé d'un acide α-amino, ou -(CH₂)_{n'}-X, où X est un groupe fonctionnel pour le couplage du ligand à une biomolécule et n' est compris entre 1 et 10 ; R₁ ou R₂ est un groupe alkoxyle C₁₋₄, et le R₁ ou R₂ restant est H, un alkyle, un groupe accepteur d'électron, ou éventuellement -(CH₂)_{n'}-X si A n'est pas -(CH₂)_{n'}-X ; A' ou A" est H, un alkyle, un groupe accepteur d'électron, ou éventuellement-(CH₂)_{n'}-X si A, R1, ou R₂ n'est pas -(CH₂)_{n'}-X; n est 1 ou 2 ; et PG est un groupe soufre protecteur.

4. Un ligand tel que défini dans l'une quelconque des revendications 1 à 3, où X est choisi parmi le groupe comprenant un carbonyle, un ester actif, un isocyanate, un isothiocyanate, un imidate, un maléimide ou un centre électrophile activé tel que C=C, un halocarbonyle, un halosulfonyle, et un haloacétyle.

5. Un ligand tel que défini dans l'une quelconque des revendications 1 à 4, où PG ou PG₁ et/ou PG₂ est/sont choisis parmi le groupe comprenant l'éthoxyéthyle, le méthoxyméthyle,le tétrahydropyranyle et le tétrahydrofuranyle substitué ou non substitué, l'acétamidoalkyle, et le S-acyle tel que le S-alkanoyle, le S- benzoyle, et les groupes benzoyle S-substitués.

6. Un ligand selon l'une quelconque des revendications 1 à 5 couplé à une biomolécule.

7. Une composition de diagnostique utile pour l'administration à un animal à sang chaud, comprenant :
une quantité efficace du point de vue diagnostique d'un ligand conforme à l'une quelconque des revendications 1 à 5, dans laquelle le ligand est couplé à une biomolécule et où le ligand est complexé avec un nucléide radioactif choisi parmi 99^{m}Tc ¹¹¹In, et ⁶²Cu ; et
un vecteur acceptable du point de vue pharmaceutique.

8. Une composition thérapeutique utile pour l'administration à un animal à sang chaud, comprenant :
une quantité efficace du point de vue thérapeutique d'un ligand conforme à l'une quelconque des revendications 1 à 5, dans laquelle le ligand est couplé à une biomolécule et où le ligand est complexé avec un nucléide radioactif choisi parmi ¹⁸⁶Re, ¹⁸⁸Re, ⁶⁷Cu ⁹⁰Y; et ⁶⁰Co ; et
un vecteur acceptable du point de vue pharmaceutique.

9. Un kit de diagnostique ou thérapeutique comprenant un ligand couplé selon la revendication 6 et un nucléide radioactif.
